# EUROPEAN PATENT SPECIFICATION

(11) **EP 1 695 720 B1**
(45) Date of publication and mention of the grant of the patent: **29.10.2008**
(21) Application number: 06251088.8
(22) Date of filing: 28.02.2006
(51) Int. Cl.: A61L 9/14, A61L 12/12, E03D 9/00, B65D 83/00

(54) **Air freshener for ceiling-mounting**
Luftauffrischer zur Unterdeckenmontage
Purificateur d'air pour le montage au plafond

(30) Priority: 28.02.2005 GB 0504127
(43) Date of publication of application: 30.08.2006
(73) Proprietor: KENNEDY HYGIENE PRODUCTS LTD, Uckfield, East Sussex TN22 1YA (GB)
(72) Inventor: West, Gavin Lloyd, Brighton East Sussex BN41 1LS (GB); Pentland, Michael Anthony, East Grinstead West Sussex RH19 1SX (GB)
(74) Representative: Bayliss, Geoffrey Cyril

(56) References cited:
- EP-A- 0 485 134
- WO-A-03/014628
- US-A- 4 830 791
- US-A- 5 884 808
- US-B1- 6 267 297

## Description

This invention relates to air fresheners for dispensing fragrances to counteract both washroom and tobacco malodours.

Air fresheners exist which have aerosol cans containing a supply of fragrance to be dispensed, a microprocessor controlled motorised unit for actuating the aerosol can to dispense a volume of fragrance at predetermined time intervals and a display indicating relevant information including the length of time left before the aerosol can will be exhausted. The microprocessor control system for the air freshener is mounted in an upper part of the air freshener and the display is located on an upper part of the front wall of the housing for the air freshener. Such air fresheners are usually mounted on a vertical wall above head height to make the dispensing of the fragrance from the air freshener as effective as possible throughout the space in which the freshener is located. The information display screen is also located towards the upper end of the air freshener convenient for the microprocessor control from which it receives information but this can result in the screen being difficult to read from below.

US-A-6267297 discloses an automatic air freshener comprising a housing having an upright base, an aerosol can containing a supply of fragrance for discharge in a spray or jet from a horizontally acting nozzle at the upper end of the can, and a battery powered mechanism for causing the aerosol to discharge periodically from the nozzle. A front cover is hinged to the base and has an upper port in line with the discharge nozzle of the aerosol. A display panel which includes information regarding the remaining number of days left in the life of the can and also the battery life is mounted behind the cover.

This invention provides an automatic air freshener comprising an upright base for mounting on a vertical wall or other face having upper and lower housings to receive an aerosol can containing a supply of fragrance for discharge in a spray or jet from a horizontally acting nozzle at the upper end of the can, a battery powered mechanism in the upper housing for causing the aerosol to discharge periodically from the nozzle, a battery pack, a control panel, a display screen and a front cover for extending over the upper and lower housings and having an upper port in line with the discharge nozzle of the aerosol can; wherein the lower housing has a front wall in which the control panel and display screen are located; and both are angled to the vertical to face downwardly from the lower housing for convenient viewing of the information on the screen and operation of the controls when the freshener is mounted on a vertical face above head height and the part of the front cover which extends over the lower housing extends over both the control panel and the screen has a downwardly angled port in line with the screen to permit viewing of the display screen through the cover.

More specifically, the screen may be angled at between 10° to 30° to the vertical and is preferably angled at 20° to the vertical. The lower housing may have a front wall containing the screen and control panel, the front wall being angled downwardly from the vertical as aforesaid.

In one arrangement according to the invention the lower housing may contain a microprocessor for controlling operation of the motor for the discharge mechanism for the aerosol and for controlling the information displayed on the screen.

The control panel on the lower housing may comprise a plurality of push buttons for controlling the microprocessor.

In any of the above arrangements the screen may be an LCD screen.

The microprocessor may provide a number of different modes of operation reflected by the information displayed on the screen. For example the microprocessor may be programmed to display "number of days left" information or may display a screensaver or recycling graphic.

In any of the above arrangements the upper port in the front cover is flared from the aerosol nozzle towards the outer side of the cover.

Also in any of the above arrangements the lower port in the front cover is flared from the display screen towards the outer face of the front cover.

Preferably the front cover is hinged to the base of the air freshener adjacent the upper end of the base to enable the air freshener to be opened for access to the aerosol can and battery pack.

The following is a description of a specific embodiment of the invention, reference being made to the accompanying drawings in which:-
Figure 1 is a perspective view of the air freshener with a front cover of the air freshener in place showing the upper port in the front cover for the outlet for a spray or jet of fragrance from an enclosed aerosol and a screen in a port in the lower part of the cover to display information such as how many days supply of fragrance is left in the aerosol can;
Figure 2 is a front elevation view of the air freshener as shown in Figure 1;
Figure 3 is an exploded perspective view of the air freshener with the front cover off to reveal upper and lower mountings locating the aerosol can and battery pack and with a panel of the lower mounting shown separated;
Figure 4 is a perspective view of the air freshener with the front cover removed to reveal upper and lower mountings locating the aerosol can and battery pack with a control panel for a microprocessor control on the front of the lower housing and a display screen also on the front window of the lower housing for displaying information from the microprocessor including the days of supply left;
Figure 5 is a side view of the air freshener without the front cover as shown in Figure 4;
Figure 6 is a similar view to Figure 4 with the aerosol can and battery pack removed;
Figure 7 is a detailed view of an upper part of the air freshener with the upper housing removed to reveal the mechanism for actuating the aerosol to discharge the supply of fragrance; and
Figures 8 and 9 illustrate the mechanism in two different positions of movement.

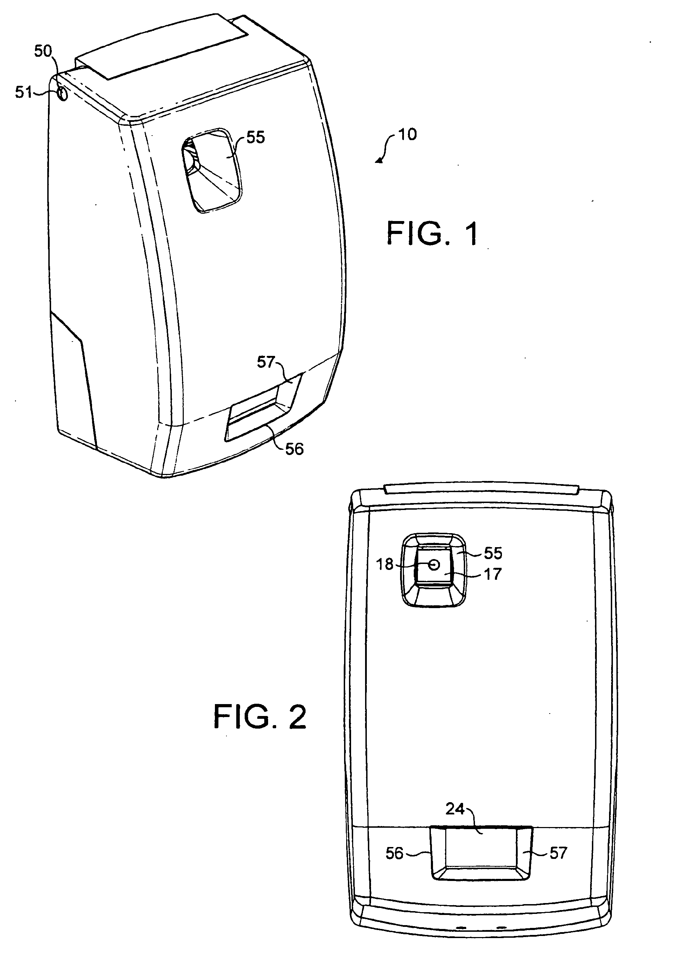

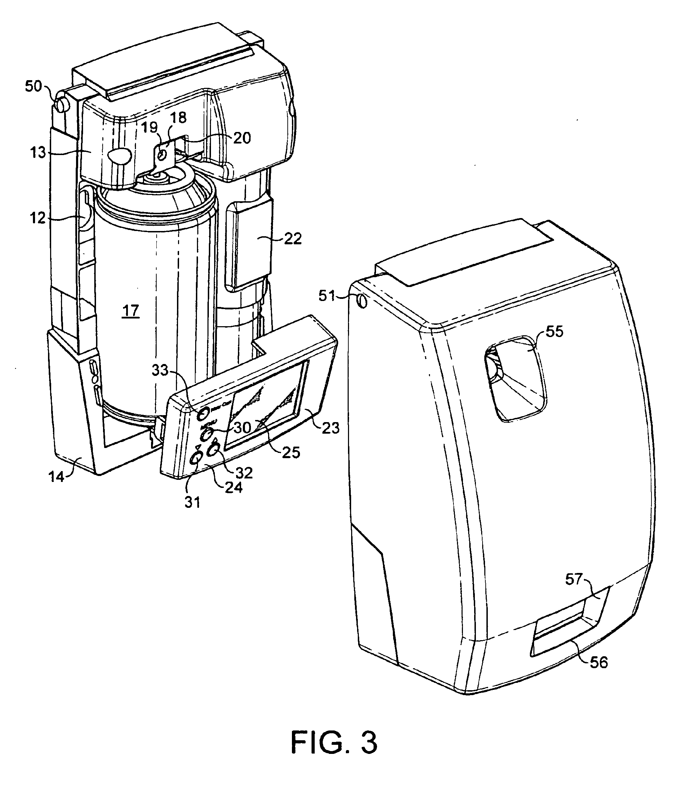

Reference is made firstly to the air freshener as shown in Figures 1 to 5 of the drawings. The air freshener comprises a housing indicated generally at 10 having a vertical base 11 formed by a plastics moulding and provided with screw apertures, one of which can be seen at 12 in Figure 3 for securing the base to a vertical wall at a location above head height to dispense fragrance into the surrounding air.

The base 11 has an upper moulded plastics housing 13 secured to the upper end of the base and a lower plastics moulded housing 14 secured to the lower part of the base. The upper wall 15 of the lower housing 14 has a semi-circular cut out 16 towards one end of the housing to receive the lower part of an aerosol can 17 for holding a supply of fragrance.

At the upper end of the can there is a spray head 18 mounted on a plunger of the valve for operating the aerosol and provided with a horizontally acting jet 19 for delivering a spray of fragrance horizontally from the head when the head is displaced downwardly against the powerful action of a return spring in the can to trigger opening of the valve of the aerosol. Depressing the head causes a predetermined quantity of fragrance to be dispersed. The head 18 of the aerosol engages in a slot 20 formed in the front part of the upper housing 13.

The upper wall 15 of the lower housing 14 has a further semi-circular cut out 21 towards the other side of the housing to receive the lower part of the battery pack 22 for powering the air freshener.

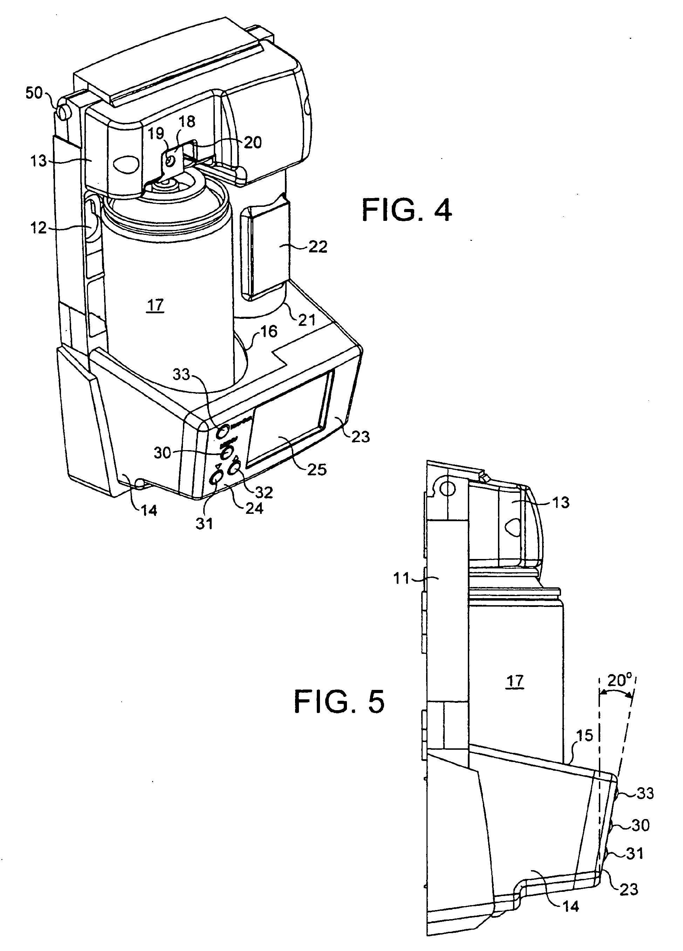

The lower housing 14 contains a microprocessor (not shown) controlling operation of the air freshener. The front wall 23 of the lower housing has a control panel located at 24 to one end of the front wall and coupled to the microprocessor and an LCD display screen 25 extending over the central region of the front wall to receive and display information from the microprocessor. As best seen in Figure 5, the front wall of the housing is inclined forwardly and downwardly from the vertical by a shallow angle of about 20° to make the screen 25 more easily viewed from below when the unit is mounted above head height on a wall. The angle may vary with housing to housing from about 10 to about 30°.

The control panel has a number of push buttons connected to the microprocessor including a button 30 to bring up the menu on the microprocessor, buttons 31 and 32 to enable the user to track through options provided by the software loaded in the microprocessor from the screen and a button 33 to signal the placement of a new aerosol can in the air freshener.

The software in the microprocessor allows the user to select the frequency of discharge of the can, e.g. every 7, 14, 20 or 30 minutes, for 12 or 24 hours a day and for 5 or 7 days a week. The software also allows the user to opt between a day by day countdown of the number of days supply left or a screensaver or other function when the user does not wish to display "days left".

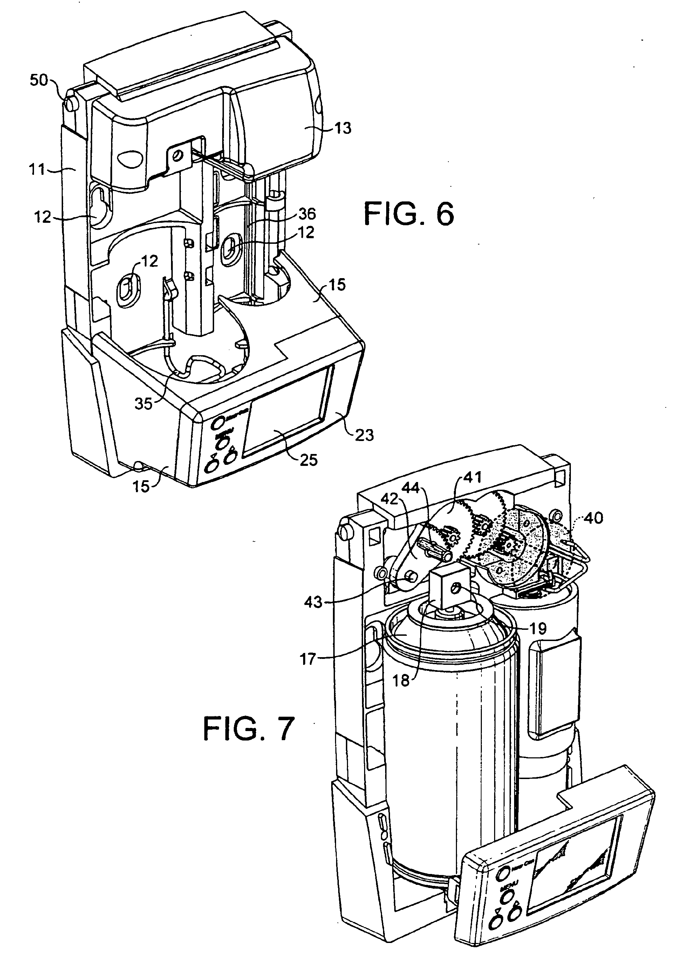

Figure 6 of the drawings is a similar view to Figure 3 but with the aerosol can and battery pack removed. In Figure 6 a pre-bent wire or rod 35 can be seen which forms a stirrup to receive and hold the aerosol can 15 in place in the lower housing. Also wiring 36 extending from the microprocessor to the motorised drive for actuating the aerosol can is shown.

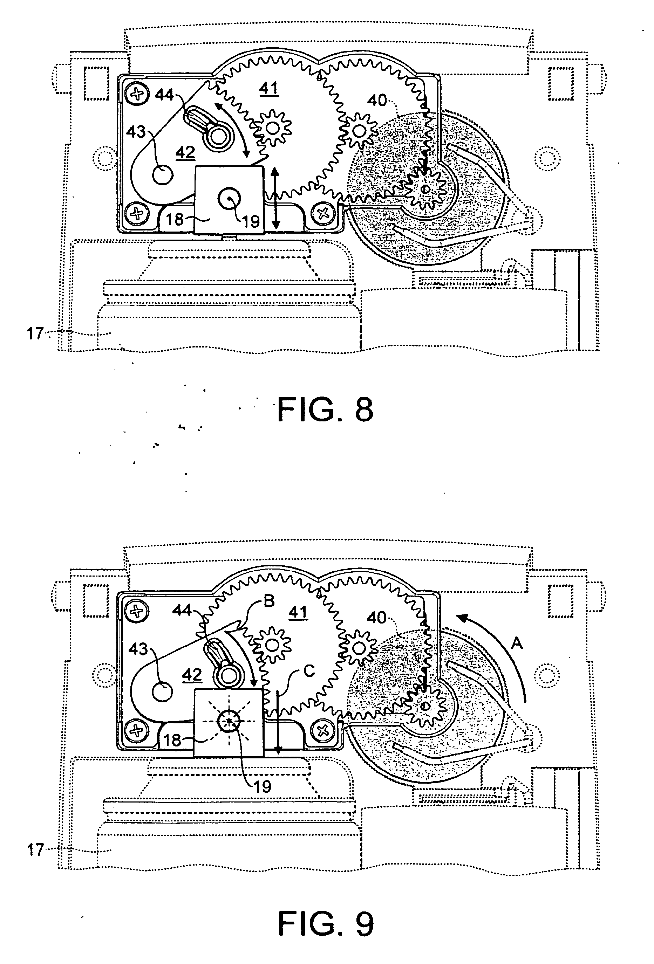

Figures 7 to 9 of the drawings shows the upper part of the base of the air freshener with the upper housing removed to reveal a small electric motor 40 mounted on the base and having a drive shaft driving through step down gearing indicated at 41 to a quadrant gear 42 supported on a pivot 43. The quadrant gear has an integral striker arm 44 which can engage the upper end of the head 18 of the aerosol can (see Figure 9) for displacing the head downwardly to open the valve of the aerosol and direct a jet of fragrance from the nozzle in the head. The motor used is of relatively low output and the step down sets of gears are required to enable the quadrant gear 42 to have the required mechanical advantage to displace the head 18. The power supply to the motor is controlled by the microprocessor circuit as indicated above. When a discharge of the requisite duration of fragrance has taken place, the microprocessor timing circuit causes the power supply to the motor to be terminated allowing the nozzle to return under its own spring force (the spring is within the nozzle can) and to return the gear drive and motor to the starting position shown in Figure 8.

The microprocessor enables the air freshener to be set to provide discharges of fragrance at required intervals such as once per hour. Since the microprocessor has a record of how many discharges the can can provide altogether, it can then compute and give an indication of the number of days supply left in the can which can be displayed on the screen. Other information can also be displayed on the screen such as the battery condition and the like.

As indicated earlier, the air freshener has a front cover. This is a moulded plastics unit which extends over the upper and lower housing and down the sides of the base. The front cover is hinged to the base on trunnions 50 projecting laterally from the sides of the base at the upper end thereof and engaging in openings 51 in the sides of the cover. A releasable latch 58 is provided on the bottom wall of the lower housing 14 to engage in an opening in the cover to hold the cover closed.

Towards the upper end of the cover at a location directly opposite the aerosol head there is a port 55 in the front wall of the cover and behind the port there is a short flared duct through which fragrance from the head 18 of the aerosol can is discharged when the head is depressed as described earlier. At the centre of the lower end of the cover there is a second port 56 which is located in register with the screen on the lower housing of the cover. An internal flared bezel 57 is integrally formed on the back of the cover to extend between the port 56 and the screen. The near part of the screen is revealed in the port leaving a part adjacent the control panel not visible when the cover is closed. The latter part of the screen is retained set up and other information not required to be observed in day to day operation of the air freshener.

## Claims

1. An automatic air freshener comprising an upright base (11) for mounting on a vertical wall or other face having upper and lower housings (13,14) to receive an aerosol can (17) containing a supply of fragrance for discharge in a spray or jet from a horizontally acting nozzle (19) at the upper end of the can, a battery powered mechanism (40-44) in the upper housing for causing the aerosol to discharge periodically from the nozzle, a battery pack (22), a control panel (24), a display screen (25), and a front cover for extending over the upper and lower housings and having an upper port (55) in line with the discharge nozzle of the aerosol; **characterised in that** the lower housing has a front wall (23) on which the control panel and display screen are located and both are angled to the vertical to face downwardly and outwardly from the lower housing for convenient viewing of the information on the screen and operation of the controls when the freshener is mounted on a vertical face above head height and the part of the front cover which extends over the lower housing extends over both the control panel and the screen and has a downwardly angled lower port (56) in line with the screen to permit viewing of the display screen through the cover.

2. An air freshener as claimed in claim 1, **characterised**
**in that** the screen (25) is an LCD screen.

3. An air freshener as claimed in claim 1 or claim 2, **characterised in that** the screen (25) and control panel are angled at between 10° to 30° to the vertical.

4. An air freshener as claimed in claim 3, **characterised in that** the screen (25) and control panel are angled at about 20° to the vertical.

5. An air freshener as claimed in any of the preceding claims, **characterised in that** the lower housing (14) has a front wall (28) containing the screen (25) and control panel, the wall being angled downwardly from the vertical.

6. An air freshener as claimed in any of claims 1 to 5, **characterised in that** the lower housing (14) contains a microprocessor for controlling operation of the motor for the discharge mechanism for the aerosol (17) and for controlling the information displayed on the screen (25), and the control panel (24) has a plurality of push buttons (30-33) for controlling the microprocessor.

7. An air freshener as claimed in any of the preceding claims, **characterised in that** the upper port (55) in the front cover (49) has a flared duct extending from the aerosol nozzle (19) towards the outer side of the cover.

8. An air freshener as claimed in any of the preceding claims, **characterised in that** the lower port (56) in the front cover (49) has a flared bezel (52) encircling the display screen (25).

9. An air freshener as claimed in any of the preceding claims, wherein the front cover (49) is hinged to the base (11) of the air freshener adjacent the upper end of the base to enable the air freshener to be opened for access to the aerosol can (17) and battery pack (22).

## Patentansprüche

1. Automatischer Lufterfrischer mit einem hochkanten Grundteil (11) zum Anbringen an einer vertikalen Wand oder einer anderen Fläche, mit einem oberen und einem unteren Gehäuse (13, 14) zum Aufnehmen einer Aerosoldose (17) enthaltend einen Vorrat eines Duftstoffes zur Abgabe in einem Spray oder Strahl aus einer horizontal wirkenden Düse (19) am oberen Ende der Dose, einem batteriebetriebenen Mechanismus (40-44) im oberen Gehäuse zum Veranlassen des Aerosols, sich periodisch aus der Düse zu entladen, einem Akkumulator (22), einem Bedienfeld (24), einem Anzeigebildschirm (25) und einer sich über das obere und das untere Gehäuse erstreckenden Vorderabdeckung, die eine obere Öffnung (55) auf gleicher Höhe mit der Aerosolauslassdüse aufweist; **dadurch gekennzeichnet, dass** das untere Gehäuse eine Vorderwand (23) aufweist, an der sich das Bedienfeld und der Anzeigebildschirm befinden, die beide zur Vertikalen abgewinkelt sind, um nach unten und nach außen aus dem unteren Gehäuse zu weisen zum günstigen Betrachten der Informationen auf dem Bildschirm und zum Bedienen der Bedienelemente, wenn der Erfrischer an einer vertikalen Fläche über Kopfhöhe angebracht ist und der sich über das untere Gehäuse erstreckende Teil der Vorderabdeckung sich sowohl über das Bedienfeld als auch über den Bildschirm erstreckt und eine nach unten abgewinkelte untere Öffnung (56) auf gleicher Höhe mit dem Bildschirm aufweist, um ein Betrachten des Anzeigebildschirms durch die Abdeckung zu erlauben.

2. Lufterfrischer nach Anspruch 1, **dadurch gekennzeichnet, dass** der Bildschirm (25) ein LCD-Bildschirm ist.

3. Lufterfrischer nach Anspruch 1 oder Anspruch 2, **dadurch gekennzeichnet, dass** der Bildschirm (25) und das Bedienfeld zwischen 10° und 30° von der Vertikalen abgewinkelt sind.

4. Lufterfrischer nach Anspruch 3, **dadurch gekennzeichnet, dass** der Bildschirm (25) und das Bedienfeld ca. 20° von der Vertikalen abgewinkelt sind.

5. Lufterfrischer nach irgendeinem der vorangehenden Ansprüche, **dadurch gekennzeichnet, dass** das untere Gehäuse (14) eine Vorderwand (28) aufweist, die den Bildschirm (25) und das Bedienfeld enthält, wobei die Wand von der Vertikalen nach unten abgewinkelt ist.

6. Lufterfrischer nach irgendeinem der Ansprüche 1 bis 5, **dadurch gekennzeichnet, dass** das untere Gehäuse (14) einen Mikroprozessor zum Steuern des Betriebs des Motors für den Abgabemechanismus für das Aerosol (17) und zum Steuern der auf dem Bildschirm (25) angezeigten Informationen enthält und das Bedienfeld (24) eine Mehrzahl von Drucktasten (30-33) zum Steuern des Mikroprozessors aufweist.

7. Lufterfrischer nach irgendeinem der vorangehenden Ansprüche, **dadurch gekennzeichnet, dass** die obere Öffnung (55) in der Vorderabdeckung (49) einen sich aufweitenden Kanal aufweist, der sich von der Aerosoldüse (19) in Richtung der Außenseite der Abdeckung erstreckt.

8. Lufterfrischer nach irgendeinem der vorangehenden Ansprüche, **dadurch gekennzeichnet, dass** die untere Öffnung (56) in der Vorderabdeckung (49) eine sich aufweitende Einfassung (52) aufweist, die den Anzeigebildschirm (25) umgibt.

9. Lufterfrischer nach irgendeinem der vorangehenden Ansprüche, wobei die Vorderabdeckung (49) am Grundteil (11) des Lufterfrischers angrenzend an das obere Ende des Grundteils angelenkt ist, um ein Öffnen des Lufterfrischers für einen Zugang zur Aerosoldose (17) und zum Akkumulator (22) zu ermöglichen.

## Revendications

1. Purificateur d'air automatique comprenant une base verticale (11) pour montage sur une paroi verticale ou une autre face, ayant des boîtiers supérieur et inférieur (13, 14) destinés à recevoir un bidon d'aérosol (17) contenant une source de parfum pour décharge en un spray ou jet depuis une buse opérant horizontalement (19) à l'extrémité supérieure du bidon, un mécanisme alimenté par pile (40 à 44) dans le boîtier supérieur pour amener l'aérosol à une décharge périodique depuis la buse, un bloc-piles (22), un panneau de commande (24), un écran d'affichage (25), et un couvercle avant destiné à s'étendre sur les boîtiers supérieur et inférieur et ayant un orifice supérieur (55) en alignement avec la buse de décharge de l'aérosol ; **caractérisé en ce que** le boîtier inférieur a une paroi avant (23) sur laquelle le panneau de commande et l'écran d'affichage sont situés et sont tous deux inclinés par rapport à la verticale pour être tournés vers le bas et vers l'extérieur depuis le boîtier inférieur afin de permettre, facilement, de voir les informations sur l'écran et d'actionner les commandes lorsque le purificateur est monté sur une face verticale au-dessus de la hauteur de la tête et **en ce que** la partie du couvercle avant qui s'étend sur le boîtier inférieur, s'étend à la fois sur le panneau de commande et l'écran et a un orifice inférieur (56) incliné vers le bas, aligné avec l'écran, pour permettre de voir l'écran d'affichage au travers du couvercle.

2. Purificateur d'air selon la revendication 1, **caractérisé en ce que** l'écran (25) est un écran à affichage à cristaux liquides.

3. Purificateur d'air selon la revendication 1 ou la revendication 2, **caractérisé en ce que** l'écran (25) et le panneau de commande sont inclinés par rapport à la verticale de 10° à 30°.

4. Purificateur d'air selon la revendication 3, **caractérisé en ce que** l'écran (25) et le panneau de commande sont inclinés par rapport à la verticale d'environ 20°.

5. Purificateur d'air selon l'une quelconque des revendications précédentes, **caractérisé en ce que** le boîtier inférieur (14) a une paroi avant (28) contenant l'écran (25) et le panneau de commande, la paroi étant inclinée vers le bas par rapport à la verticale.

6. Purificateur d'air selon l'une quelconque des revendications 1 à 5, **caractérisé en ce que** le boîtier inférieur (14) contient un microprocesseur pour commander le fonctionnement du moteur pour le mécanisme de décharge de l'aérosol (17) et pour commander les informations affichées sur l'écran (25), et **en ce que** le panneau de commande (24) a une pluralité de boutons poussoirs (30 à 33) pour commander le microprocesseur.

7. Purificateur d'air selon l'une quelconque des revendications précédentes, **caractérisé en ce que** l'orifice supérieur (55) dans le couvercle avant (49) comporte un conduit évasé s'étendant depuis la buse d'aérosol (19) vers le côté externe du couvercle.

8. Purificateur d'air selon l'une quelconque des revendications précédentes, **caractérisé en ce que** l'orifice inférieur (56) dans le couvercle avant (49) comporte une collerette évasée (52) entourant l'écran d'affichage (25).

9. Purificateur d'air selon l'une quelconque des revendications précédentes, dans lequel le couvercle avant (49) est articulé sur la base (11) du purificateur d'air en un point adjacent à l'extrémité supérieure de la base pour permettre au purificateur d'air d'être ouvert afin d'avoir accès au bidon d'aérosol (17) et au bloc-piles (22).
